(19) Europäisches Patentamt
European Patent Office
Office européen des brevets

(11) EP 1 859 779 A2

(12) **EUROPÄISCHE PATENTANMELDUNG**

(43) Veröffentlichungstag:
**28.11.2007 Patentblatt 2007/48**

(51) Int Cl.:
***A61K 8/37*** *(2006.01)* ***A61Q 17/04*** *(2006.01)*

(21) Anmeldenummer: **07108544.3**

(22) Anmeldetag: **21.05.2007**

(84) Benannte Vertragsstaaten:
**AT BE BG CH CY CZ DE DK EE ES FI FR GB GR HU IE IS IT LI LT LU LV MC MT NL PL PT RO SE SI SK TR**
Benannte Erstreckungsstaaten:
**AL BA HR MK YU**

(30) Priorität: **24.05.2006 DE 102006025057**

(71) Anmelder: **Beiersdorf AG**
**20253 Hamburg (DE)**

(72) Erfinder:
- **Eitrich, Anja**
**22587, Hamburg (DE)**
- **Sugár, Martin Dr.**
**20253 Hamburg (DE)**
- **Mundt, Claudia**
**28207, Bremen (DE)**
- **Tesch, Mirko**
**22303, Hamburg (DE)**
- **Weingarz, Yvonne**
**20251, Hamburg (DE)**

# (54) Kosmetische Lichtschutzzubereitung mit besonderer Textur

(57) Kosmetische Lichtschutzzubereitung, dadurch gekennzeichnet, dass sie
a) eine oder mehrere öllösliche UV-Filtersubstanzen,
b) eine oder mehrere Ölkomponenten mit einer Polarität ≤ 35 mN/m,
c) einen oder mehrere Salicylatester, welcher durch die Strukturformel

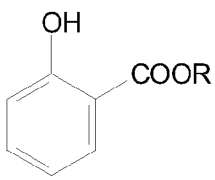

gekennzeichnet ist, worin R einen unverzweigten Alkylrest mit 5 bis 16 Kohlenstoffatomen oder einen verzweigten Alkylrest mit 9 bis 20 Kohlenstoffatomen darstellt,
d) eine oder mehrere flüchtige Substanzen, deren Verdunstungszahl zwischen 3 und 12 liegt und
e) Wasser

enthält.

EP 1 859 779 A2

## Beschreibung

Die vorliegende Erfindung betrifft kosmetische Lichtschutzzubereitungen - insbesondere kosmetische Zubereitungen vom Typ Öl-in-Wasser- mit einer besonderen Textur.

Unter Kosmetik kann man alle Maßnahmen zusammenfassen, die aus ästhetischen Gründen Veränderungen an Haut und Haaren vornehmen oder zur Körperreinigung angewendet werden. Kosmetik bedeutet also, das Körperäußere zu pflegen, zu verbessern und zu verschönern, um auf sichtbare, fühlbare und riechbare Weise sowohl den Mitmenschen als auch sich selbst zu gefallen. Schon vor Jahrtausenden wurde Kosmetik vom Menschen zu diesem Zweck angewandt. Man färbte Lippen und Gesicht, salbte sich mit wertvollen Ölen und badete in duftendem Wasser.

Während die Reinigungswirkung eines Shampoos, die Kämmbarkeitsverbesserung einer Haarspülung oder die Kräuselung des Haares durch eine Dauerwelle einfach und objektiv feststellbar sind, sind andere Wirkungen und Eigenschaften kosmetischer Produkte praktisch nicht messbar oder nur sehr individuell spürbar. Hierzu zählen zum Beispiel ein bestimmtes (belebendes, weiches, geschmeidiges, glattes etc.) Hautgefühl oder die Weichheit und Geschmeidigkeit der Haut nach der Anwendung eines Kosmetikums sowie die Fülle und Sprungkraft der Haare und dergleichen mehr. Ferner unterliegt die Erwartung der Verbraucher auch nebengeordneten Eigenschaften des Produktes. Hier sind insbesondere der Duft und die Farbe des Kosmetikums sowie seine Verpackung, der Preis, der Hersteller und die Werbeaussage zu nennen.

Eine für den Verbraucher sehr wesentliche, dabei aber nur schwer quantitativ messbare Eigenschaft kosmetischer Produkte ist ihre Textur. Unter dem Begriff "Textur" werden diejenigen Eigenschaften eines Kosmetikums verstanden, die auf den Gefügebau der Zubereitung zurückgehen, durch Tast- und Berührungssinne empfunden und ggf. in mechanischen oder rheologischen Fließeigenschaften ausgedrückt werden können. Die Textur kann insbesondere mittels Sensorik getestet werden. Die gegebenenfalls mit Hilfe von Zusatzstoffen beeinflussbare Textur kosmetischer Produkte ist für den Verbraucher von nahezu gleicher Bedeutung wie deren objektiv feststellbaren Wirkungen.

Mit dem Begriff "Sensorik" wird die wissenschaftliche Disziplin bezeichnet, die sich mit der Bewertung von kosmetischen Zubereitungen auf Grund von Sinneseindrücken befasst. Die sensorische Beurteilung eines Kosmetikums erfolgt anhand der visuellen, olfaktorischen und haptischen Eindrücke.

- *Visuelle Eindrücke:* alle mit dem Auge wahrnehmbaren Merkmale (Farbe, Form, Struktur).
- *Olfaktorische Eindrücke:* alle beim Einziehen von Luft durch die Nase wahrnehmbaren Geruchseindrücke, die häufig in Anfangsgeruch (Kopfnote), Hauptgeruch (Mittelnote, Körper) und Nachgeruch (Ausklang) differenziert werden können. Auch die erst bei der Anwendung freigesetzten flüchtigen Stoffe tragen zum olfaktorischen Eindruck bei.
- *Haptische Eindrücke:* alle Empfindungen des Tastsinns, die vornehmlich Gefüge und Konsistenz des Produktes betreffen.

Die sensorische Analyse macht von der Möglichkeit Gebrauch, den sensorischen Gesamteindruck eines Produktes integral zu erfassen. Nachteile der sensorische Analyse sind die Subjektivität des Eindrucks, eine leichte Beeinflussbarkeit der Prüfpersonen und die dadurch bedingte starke Streuung der Ergebnisse. Diesen Schwächen begegnet man heute durch den Einsatz von Gruppen geschulter Prüfpersonen, gegenseitige Abschirmung der Prüfer sowie statistische Auswertung der meist zahlreichen Analysendaten.

Die am häufigsten in der Forschung und Entwicklung genutzte Methode der sensorischen Analyse ist die Unterschiedsprüfung. Die Aufgabe beschränkt sich hierbei üblicherweise auf die Erkennung eines von mehreren Proben oder von einer Kontrollprobe abweichenden Musters. Während bei Unterschiedsprüfungen innerhalb eines Tests nur zwei Proben miteinander verglichen werden, ist bei der Rangordnungsprüfung eine Reihenfolge von drei und mehr Proben festzulegen und zwar in der Regel nach Intensität, Qualität, Beliebtheit oder Ähnlichkeit mit einer Vergleichsprobe. Diese (einfache) Methode eignet sich z. B. für eine Vorauswahl von Proben in der Produkt-Optimierung und wird auch häufig in der Marktforschung eingesetzt.

Die Textur einer kosmetischen Zubereitung lässt sich hilfsweise auch anhand ihrer rheologischen Fließeigenschaften charakterisieren.

Unter dem Begriff "Viskosität" versteht man die Eigenschaft einer Flüssigkeit, der gegenseitigen laminaren Verschiebung zweier benachbarter Schichten einen Widerstand (Zähigkeit, innere Reibung) entgegenzusetzen. Man definiert diese so genannte dynamische Viskosität nach $\eta = \tau / D$ als das Verhältnis der Schubspannung zum Geschwindigkeitsgradienten senkrecht zur Strömungsrichtung.

Während die graphische Darstellung des Fließverhaltens Newtonscher Flüssigkeiten bei konstanter Temperatur eine Gerade ergibt, zeigen sich bei den so genannten nichtnewtonschen Flüssigkeiten in Abhängigkeit von der jeweiligen Schubspannung $\tau$ oft erhebliche Abweichungen. In diesen Fällen lässt sich die so genannte scheinbare Viskosität bestimmen, die zwar nicht der Newtonschen Gleichung gehorcht, aus der sich jedoch durch graphische Verfahren die wahren Viskositätswerte ermitteln lassen.

Unter dem Begriff Fließgrenze wird die kleinste Schubspannung verstanden, oberhalb derer ein plastischer

Stoff sich rheologisch wie eine Flüssigkeit verhält (DIN 1342-1: 1983-10). Die Bestimmung der Fließgrenze erfolgt durch Aufnahme einer Fließkurve (DIN 53019: 1980-05; DIN 53214: 1982-02). Der erhaltene Wert hängt stark von der Zeitskala (Belastungsrate) ab, die der Messung zugrunde liegt. Die Fließkurve ist die graphische Darstellung des Zusammenhangs zwischen Schubspannung und Geschwindigkeitsgefälle D für eine einer Schichtenströmung unterworfenen Flüssigkeit oder für einen plastischen Stoff oberhalb der Fließgrenze. Die Messung von Fließkurven erfolgt üblicherweise in Rotationsviskosimetern. Bei drehzahlgesteuerten Systemen wird unter Vorgabe eines kontinuierlich oder schrittweise variierten Geschwindigkeitsgefälles das resultierende Drehmoment gemessen und die dazu proportionale Schubspannung errechnet. Bei schubspannungskontrollierten Systemen gilt der umgekehrte Sachverhalt. Aus der Fließkurve können die Viskosität als Funktion des Geschwindigkeitsgefälles errechnet, Fließgrenzen bestimmt und das Fließverhalten charakterisiert werden.

Kosmetische Formulierungen weisen häufig ein pseudoplastisches Fließverhalten mit mehr oder minder ausgeprägter Thixotropie auf, die es dem Verbraucher erleichtert, solche Produkte zu applizieren. Außerdem bestimmen die Stärke und Art der Pseudoplastizität sowie die Thixotropie auch die Verteilung auf der Haut und das Hautgefühl nach dem Eincremen: Beispielsweise kann eine thixotrope O/W-Formulierung nach dem Verteilen wieder eine (fühlbare) Struktur auf der Haut aufbauen.

Zum Schutz gegen die schädigende Wirkung des ultravioletten Teils der Sonnenstrahlung auf die Haut sind zahlreiche Verbindungen bekannt, die in eine kosmetische Lichtschutzzubereitung eingearbeitet werden können. Dabei handelt es sich beispielsweise um Derivate des 3-Benzylidencamphers, der 4-Aminobenzoesäure, der Zimtsäure, der Salicylsäure, des Benzophenons, des Triazins sowie Dibenzoylmethanderivate und bestimmte wasserlösliche, sulfonierte UV-Filtersubstanzen und Merocyanine.

Um einen optimalen UV-Schutz zu gewährleisten, sollten diese UV-Filtersubstanzen selbstverständlich in gelöster Form vorliegen, wozu - sofern es sich um öllösliche Filtersubstanzen handelt - hohe Mengen bestimmter Ölkomponenten eingesetzt werden müssen.

Natürlich ist dem Fachmann eine Vielzahl von Möglichkeiten bekannt, stabile Zubereitungen zur kosmetischen Anwendung zu formulieren, beispielsweise in Form von Cremes und Salben, die im Bereich von Raum- bis Hauttemperatur streichfähig sind, oder als Lotionen und Milche, die in diesem Temperaturbereich eher fließfähig sind.

Ein Nachteil des Standes der Technik ist aber, dass übliche Lichtschutzformulierungen einen meist klebrigen und/oder fettigen Film auf der Haut hinterlassen, was vom Anwender als unangenehm empfunden wird und im schlimmsten Fall dazu führen kann, dass das Sonnenschutzmittel zu wenig oder gar nicht mehr verwendet wird. Dieser Effekt wird durch den Zusatz von Glycerin noch verstärkt.

Eine Aufgabe der vorliegenden Erfindung war es daher, Lichtschutzzubereitungen zu finden, welche neben den für diese Art von Kosmetika üblichen Kriterien wie optimaler UV-Schutz, Verträglichkeit, Lagerstabilität und dergleichen auch für den Verbraucher wesentliche, bisher nicht gekannte kosmetische Leistungen bieten. Insbesondere sollten sich die gesuchten Zubereitungen für eine Verwendung im Körperpflegebereich, d. h. bei einer Anwendung auf dem ganzen Körper (und damit in Abgrenzung von einer reinen Gesichtspflegeanwendung in relativ großer Menge) eignen, dabei aber gleichzeitig für eine Anwendung im Gesichtsbereich sensorisch attraktiv sein. Dies äußert sich insbesondere in einer guten Verteilbarkeit, einem schnellen Einzugsvermögen sowie in einer gehaltvollen und pflegenden Anmutung.

Überraschend hat sich gezeigt, dass eine
kosmetische Lichtschutzzubereitung, dadurch gekennzeichnet, dass sie

a) eine oder mehrere öllösliche UV-Filtersubstanzen,
b) eine oder mehrere Ölkomponenten mit einer Polarität ≤ 35 mN/m,
c) einen oder mehrere Salicylatester, welcher durch die Strukturformel

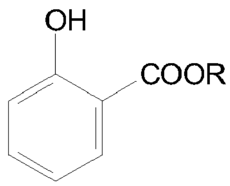

gekennzeichnet ist, worin R einen unverzweigten Alkylrest mit 5 bis 16 Kohlenstoffatomen oder einen verzweigten Alkylrest mit 9 bis 20 Kohlenstoffatomen darstellt,
d) eine oder mehrere flüchtige Substanzen, deren Verdunstungszahl zwischen 3 und 12 liegt und
e) Wasser
enthält,

diese Aufgaben zu lösen vermögen.

Die erfindungsgemäßen Zubereitungen stellen in jeglicher Hinsicht überaus befriedigende Präparate dar. Es war für den Fachmann nicht vorauszusehen gewesen, dass sich die Lichtschutzzubereitungen im Sinne der vorliegenden Erfindung insbesondere sehr leicht auf der Haut verteilen lassen, dabei schnell einziehen und wenig kleben und trotz eines sehr geringen Rückstands dennoch sehr gehaltvoll und pflegend sein würden. Die erfindungsgemäßen Formulierungen zeichnen sich ferner dadurch aus, dass sie ein besonderes Frischegefühl auf der Haut hervorrufen, lichtstrapazierte Haut pflegen, vom Sonnenbaden gereizte Haut beruhigen und feuchtigkeitsspendend wirken.

Die besondere Textur der erfindungsgemäßen kosmetischen Zubereitungen lässt sich hilfsweise anhand ihrer rheologischen Fließeigenschaften charakterisieren. Dazu werden Fließkurven der Zubereitungen mit einem SR-2000 der Fa. Rheometric Scientific (jetzt Fa. TA-Instruments) aufgezeichnet. Dieses Gerät ist ein schubspannungsgesteuertes Rheometer mit einem luftgelagerten Transducer. Das Meßsystem besteht aus einem parallelen Plattenmeßsystem (sog. Platte/Platte-Anordnung) mit einem Durchmesser von 25 mm, wobei die untere Platte mit einem Peltierelement temperierbar ist. Die Messung wird bei einer Messtemperatur von 25 °C durchgeführt. Als Meßmethode wird eine lineare Schubspannungs-Zeit-Rampe mit einer Belastungsrate von 40 Pa/min beginnend bei 0 Pa gewählt.

Es ist vorteilhaft im Sinne der vorliegenden Erfindung, die öllöslichen UV-Filtersubstanzen (eine oder mehrere Verbindungen) aus den folgenden Gruppen zu wählen:

- Dibenzoylmethanderivate, insbesondere das 4-(tert.-Butyl)-4'-methoxydibenzoylmethan (CAS-Nr. 70356-09-1), welches von Givaudan unter der Marke Parsol® 1789 und von Merck unter der Handelsbezeichnung Eusolex® 9020 verkauft wird.
- Benzoxazol-Derivate, wie z. B. das 2,4-bis-[5-1(dimethylpropyl)benzoxazol-2-yl-(4-phenyl)-imino]-6-(2-ethylhexyl)-imino-1,3,5-triazin mit der CAS Nr. 288254-16-0, welches bei 3V Sigma unter der Handelsbezeichnung Uvasorb® K2A erhältlich ist.
- Hydroxybenzophenone, z. B. der 2-(4'-Diethylamino-2'-hydoxybenzoyl)-benzoesäurehexylester (auch: Aminobenzophenon), welcher unter der Handelsbezeichnung Uvinul® A Plus bei der Fa. BASF erhältlich ist.
- Triazinderivate, wie z. B. 2,4-Bis-{[4-(2-Ethyl-hexyloxy)-2-hydroxy]-phenyl}-6-(4-methoxyphenyl)-1,3,5-triazin (INCI: Bis-Ethylhexyloxyphenol Methoxyphenyl Triazin), welches unter der Handelsbezeichnung Tinosorb® S bei der CIBA-Chemikalien GmbH erhältlich ist; Dioctylbutylamidotriazon (INCI: Diethylhexyl Butamido Triazone), welches unter der Handelsbezeichnung UVASORB HEB bei Sigma 3V erhältlich ist; 4,4',4"-(1,3,5-Triazin-2,4,6-triyitriimino)-tris-benzoesäure-tris(2-ethylhexylester), auch: 2,4,6-Tris-[anilino-(p-carbo-2'-ethyl-1'-hexyloxy)]-1,3,5-triazin (INCI: Ethylhexyl Triazone), welches von der BASF Aktiengesellschaft unter der Warenbezeichnung UVINUL® T 150 vertrieben wird; 2-[4,6-Bis(2,4-dimethylphenyl)-1,3,5-triazin-2-yl]-5-(octyloxy)phenol (CAS Nr.: 2725-22-6).
- Benzotriazole, wie z. B. 2,2'-Methylen-bis-(6-(2H-benzotriazol-2-yl)-4-(1,1,3,3-tetramethylbutyl)-phenol) (INCI: Methylene Bis-Benztriazolyl Tetramethylbutylphenol), welches z. B. unter der Handelsbezeichnung Tinosorb® M bei der CIBA-Chemikalien GmbH erhältlich ist.
- 3-Benzylidencampher-Derivate, vorzugsweise 3-(4-Methylbenzyliden)campher, 3-Benzylidencampher;
- 4-Aminobenzoesäure-Derivate, vorzugsweise 4-(Dimethylamino)-benzoesäure(2-ethylhexyl)ester, 4-(Dimethylamino)benzoesäureamylester;
- Ester der Benzalmalonsäure, vorzugsweise 4-Methoxybenzalmalonsäuredi(2-ethylhexyl)-ester;
- Ester der Zimtsäure, vorzugsweise 4-Methoxyzimtsäure(2-ethylhexyl)ester (Octylmethoxycinnamat), 4-Methoxyzimtsäureisopentylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- an Polymere gebundene UV-Filter;
- Ethylhexyl-2-cyano-3,3-diphenylacrylat (Octocrylen), welches von BASF unter der Bezeichnung Uvinul® N 539 T erhältlich ist;
- Dimethicondiethylbenzalmalonat (INCI: Polysilicone-15), welches unter dem Handelsnamen Parsol SLX von der Firma DSM erhältlich ist;
- 2-Ethylhexyl-2-hydroxybenzoat (INCI: Ethylhexylsalicylat);
- Homomenthylsalicylat (INCI: Homosalate) sowie
- 2-(2H-benzotriazol-2-yl)-4-methyl-6-[2-methyl-3-[1,3,3,3-tetramethyl-1-[(trimethylsilyl)oxy]disiloxanyl]propyl]-phenol (CAS-Nr.: 155633-54-8) mit der INCI-Bezeichnung Drometrizole Trisiloxane, welches unter dem Handelsnamen Mexoryl XL von der Fa. Chimex erhältlich ist.

Kosmetische Lichtschutzzubereitungen im Sinne der vorliegenden Erfindung enthalten - bezogen auf ihr jeweiliges Gesamtgewicht - vorteilhaft 1 bis 30 Gew.-%, insbesondere 5 bis 20 Gew.-% und ganz besonders 10 bis 15 Gew.-%, an öllöslichen UV-Filtersubstanzen (eine oder mehrere Verbindungen).

Es ist vorteilhaft ferner im Sinne der vorliegenden Erfindung, wenn die Lichtschutzzubereitungen - bezogen

auf ihr jeweiliges Gesamtgewicht - zwischen 0,1 und 2 Gew.-% Octylmethoxycinnamat enthalten.

Die erfindungsgemäßen Zubereitungen können ferner neben den öllöslichen vorteilhaft auch wasserlösliche UV-Filtersubstanzen enthalten, insbesondere solche (eine oder mehrere Verbindungen), welche aus den folgenden Gruppen gewählt werden:

- Phenylen-1,4-bis-(2-benzimidazyl)-3,3'-5,5'-tetrasulfonsäure und ihre Salze, besonders die entsprechenden Natrium-, Kalium- oder Triethanolammonium-Salze, insbesondere das Phenylen-1,4-bis-(2-benzimidazyl)-3,3'-5,5'-tetrasulfonsäure-bis-natriumsalz mit der INCI-Bezeichnung Disodium Phenyl Dibenzimidazol Tetrasulfonat (CAS-Nr.: 180898-37-7), welches beispielsweise unter der Handelsbezeichnung Neo Heliopan AP bei Symrise erhältlich ist;
- Salze der 2-Phenylbenzimidazol-5-sulfonsäure, wie ihr Natrium-, Kalium- oder ihr Triethanolammonium-Salz sowie die Sulfonsäure selbst mit der INCI Bezeichnung Phenylbenzimidazole Sulfonsäure (CAS.-Nr. 27503-81-7), welches beispielsweise unter der Handelsbezeichnung Eusolex 232 bei Merck oder unter Neo Heliopan Hydro bei Symrise erhältlich ist;
- 1,4-di(2-oxo-10-Sulfo-3-bornylidenmethyl)-Benzol (auch: 3,3'-(1,4-Phenylendimethylene)-bis-(7,7-dimethyl-2-oxo-bicyclo-[2.2.1]hept-1-ylmethan Sulfonsäure) und dessen Salze (besonders die entsprechenden 10-Sulfato-verbindungen, insbesondere das entsprechende Natrium-, Kalium- oder Triethanolammonium-Salz), das auch als Benzol-1,4-di(2-oxo-3-bornylidenmethyl-10-sulfonsäure) bezeichnet wird. Benzol-1,4-di(2-oxo-3-bornylidenmethyl-1 0-sulfonsäure) hat die INCI-Bezeichnung Terephtalidene Dicampher Sulfonsäure (CAS.-Nr.: 90457-82-2) und ist beispielsweise unter dem Handelsnamen Mexoryl SX von der Fa. Chimex erhältlich;
- Sulfonsäure-Derivate des 3-Benzylidencamphers, wie z. B. 4-(2-Oxo-3-bornylidenmethyl)-benzolsulfonsäure, 2-Methyl-5-(2-oxo-3-bornylidenmethyl)sulfonsäure und deren Salze.

Die Auswahl der UV-Filtersubstanzen (öllösliche und ggf. wasserlösliche) erfolgt vorzugsweise in der Art, dass ein optimaler UV-Schutz sowohl im UV-A als auch im UV-B-Bereich erzielt wird. Unter "optimalem UV-Schutz" ist im Sinne der vorliegenden Erfindung zu verstehen, dass die Abschwächung des ultravioletten Teils der Sonnenstrahlung über den gesamten betroffenen Wellenlängenbereich möglichst gleichmäßig erfolgt ("kastenförmiges" Absorptionsspektrum).

Dies lässt sich beispielsweise mit Hilfe der UVA-Balance charakterisieren, welche definiert ist als:

$$UVA-Balance = \frac{PPD_{in\,vitro} - 1}{SPF_{in\,vivo} - 1} \cdot 100$$

Der PPD-Wert wird üblicherweise *in vitro* bestimmt, beispielsweise wie bei Wendel *et al.* beschrieben (Wendel V. et al., 2003, The influence of pre-irradiation on the predictability of in vivo UVA protection with a new in vitro method, Photodermatol Photoimmunol Photomed, 19:93-97). Hilfsweise kann der PPD-Wert alternativ auch *in vivo* bestimmt werden. Der Wert für den SPF (Sun Protection Factor, auch "LSF" für Lichtschutzfaktor genannt) wird gemäß Colipa *in vivo* bestimmt.

Besonders vorteilhafte Zubereitungen im Sinne der vorliegenden Erfindung weisen eine UVA-Balance von mehr als 22, insbesondere mehr als 30, ganz besonders bevorzugt mehr als 40 auf.

Dementsprechend ist es erfindungsgemäß vorteilhaft, wenn das Verhältnis der eingesetzten UV-A-Filtermengen zu den eingesetzten UV-B-Filtermengen ausreichend hoch ist. Bevorzugte Mischungsverhältnisse von UV-A-Filtern zu UV-B-Filtern liegen im Bereich von 0,1 bis 4 (bezogen auf die Massen der eingesetzten UV-Filter). Besonders bevorzugte Mischungsverhältnisse von UV-A-Filtern zu UV-B-Filtern liegen im Bereich von 0,2 bis 2,5 (bezogen auf die Massen der eingesetzten UV-Filter). Ganz besonders bevorzugte Mischungsverhältnisse von UV-A-Filtern zu UV-B-Filtern liegen im Bereich von 0,3 bis 1 (bezogen auf die Massen der eingesetzten UV-Filter). Zur Gruppe der UV-A-Filter gehören u. a. die oben beschriebenen Dibenzoylmethanderivate, die Phenylen-1,4-bis-(2-benzimidazyl)-3,3'-5,5'-tetrasulfonsäure und ihre Salze, das 1,4-di(2-oxo-10-Sulfo-3-bornylidenmethyl)-Benzol und dessen Salze, das 2,4-bis-[5-1 (dimethylpropyl)benzoxazol-2-yl-(4-phenyl)-imino]-6-(2-ethylhexyl)-imino-1,3,5-triazin oder der 2-(4'-Diethylamino-2'-hydoxybenzoyl)-benzoesäurehexylester (auch: Aminobenzophenon). Zur Gruppe der UV-B-Filter gehören u. a. die oben beschriebenen Salze der 2-Phenylbenzimidazol-5-sulfonsäure, Dioctylbutylamidotriazon, Ethylhexyl Triazone, 3-Benzylidencampher-Derivate, Ester der Zimtsäure, Octocrylene oder an Polymere gebundene UV-B-Filter.

Weitere besonders vorteilhafte Zubereitungen im Sinne der vorliegenden Erfindung, die sich durch eine hohe bzw. sehr hohe UVA-Balance auszeichnen, enthalten bevorzugt so genannte organische Breitbandfilter in Konzentrationen von 0,5 Gew.-% (bezogen auf das Gesamtgewicht der Zubereitung) und mehr. Zur Gruppe der Breitbandfilter gehören beispielsweise unsymmetrisch substituierte s-Triazin-Verbindungen, wie z. B. das 2,4-Bis-{[4-(2-ethyl-hexyloxy)-2-hydroxy]-phenyl}-6-(4-methoxyphenyl)-1,3,5-triazin, bestimmte Benzophenone, wie z. B. das 2-Hydroxy-4-methoxy-benzophenon oder das 2,2'-Methylen-bis-(6-(2H-benzotriazol-2-yl)-4-(1,1,3,3-tetramethylbutyl)-phenol). Ferner vorteilhaft ist auch der Einsatz von pigmentären, anorganischen UV-Filtern in Konzentrationen von 0,5 Gew.-% oder mehr. Zu dieser Gruppe gehören insbesondere Metalloxide wie Titandioxid und Zinkoxid, die sowohl Schutz vor UVBals auch UVA-Strahlung bieten.

Erfindungsgemäß vorteilhaft können auch organische Pigmente eingesetzt werden. Dabei ist es erfindungsgemäß besonders bevorzugt, wenn als organische Pigmente 2,4,6-Tribiphenyl-4-yl-1,3,5-triazin und/oder 2,2'-Methylen-bis-(6-(2H-benzotriazol-2-yl)-4-(1,1,3,3-tetramethylbutyl)-phenol) eingesetzt werden.

Die anspruchsgemäßen Ölkomponenten (eine oder mehrere Verbindungen) werden vorzugsweise aus der folgenden Gruppe gewählt:

| **Hersteller** | **Handelsname** | **INCI-Name** |
|---|---|---|
| Cognis | Cetiol CC | Dicaprylylcarbonat |
| Cognis | Cetiol OE | Dicaprylylether |
| Stearinerie Dubois Fils | DUB VCI 10 | Isodecyl Neopentanoate |
| Lipo Chemicals Inc | Liponate TDTM | Tridecyl Trimellitate |
| Wacker | Wacker AK 100 | Dimethicone |
| Cognis | Isopropylpalmitat | Isopropylpalmitat |
| Cognis | Eutanol G | Octyldodecanol |
| Bayer AG, Dow Corning | Silikonöl VP 1120 | Phenyl Trimethicone |
| Henkel Cognis | Isopropylstearat | Isopropyl Stearate |
| WITCO, Goldschmidt | Finsolv TN | C12-15 Alkyl Benzoate |
| Dr. Straetmans | Dermofeel BGC | Butylene Glycol Dicaprylate/Dicaprate |
| Uniqema, Huels | Miglyol 812 | Caprylic/Capric Triglyceride |
| Dow Corning | DC Fluid 345 | Cyclomethicon |
| Cognis | Cetiol B | Dibutyl Adipate |
| Condea Augusta S.P.A. | Cosmacol ELI | C12-13 Alkyl Lactate |
| Condea Augusta S.P.A. | Cosmacol ETI | Di-C12/13 Alkyl Tartrate |
| Cognis | Myritol331 | Cocoglycerides |
| Symrise | Corapan TQ | Diethylhexylnaphthalat |
| ISP | X-Tend 226 | 2-Phenylethylbenzoat |
| Finetex | Finsolv Sun | Phenylethylbenzoat |
| Ajinomoto | ELDEW SL-205 | Isopropyl Lauroyl Sarkosinat |

Erfindungsgemäß besonders bevorzugt sind die Ölkomponenten mit den INCI-Bezeichnungen Octyldodecanol, C12-15 Alkyl Benzoate, Butylene Glycol Dicaprylate/Dicaprate, Caprylic/Capric Triglyceride, Cyclomethicon, Dicaprylylcarbonat, 2-Phenylethylbenzoat, Isopropyl Lauroyl Sarkosinat, jeweils einzeln oder in Kombinationen miteinander.

Es kann vorteilhaft im Sinne der vorliegenden Erfindung sein, wenn die Ölphase der Zubereitung neben der erfindungsgemäßen Ölkomponenten weitere Ölkomponenten enthält.

Erfindungsgemäß besonders bevorzugte weitere Ölkomponenten sind Paraffinöl (in Konzentrationen von 2 bis 9 Gew.-%), Mineralöl (1 bis 5 Gew.-%), Isohexadecan (2 bis 8 Gew.-%), Cyclohexasiloxan (1 bis 5 Gew.-%), Coco Caprylate/Caprate (2 bis 10 Gew.-%), Ethylhexylstearat (1 bis 6 Gew.-%) und/oder Myristylmyristat (0,5 bis 5 Gew.-%), wobei die Konzentrationsangaben jeweils auf das Gesamtgewicht der Zubereitung bezogen sind.

Vorteilhafte Salicylatester im Sinne der vorliegenden Erfindung sind beispielsweise die Substanzen mit den INCI-Bezeichnungen C12-C15 Alkylsalicylate (unter der Handelsbezeichnung Dermol NS bei der Fa. Alzo erhältlich) und Butyloctyl Salicylate (unter der Handelsbezeichnung Hallbrite BHB bei der Fa. RTD HallStar erhältlich).

Unter den Salicylatestern sind diejenigen erfindungsgemäß bevorzugt, für die R in der oben genannten Strukturformel einen unverzweigten Alkylrest darstellt. Ganz besonders bevorzugt sind Salicylatester für die R in der oben genannten Strukturformel einen unverzweigten Alkylrest mit 10 bis 16 Kohlenstoffatomen darstellt. Erfindungsgemäß besonders bevorzugt ist Tridecylsalicylat, welches unter der Handelsbezeichnung Cosmacol ESI bei der Fa. Sasol erhältlich ist.

Kosmetische Lichtschutzzubereitungen im Sinne der vorliegenden Erfindung enthalten - bezogen auf ihr jeweiliges Gesamtgewicht - vorteilhaft 0,01 bis 20 Gew.-%, insbesondere 0,5 bis 10 Gew.-% und ganz besonders 1 bis 7 Gew.-%, an Salicylatestern (eine oder mehrere Verbindungen).

Die erfindungsgemäßen Zubereitungen enthalten mindestens eine flüchtige Substanz. Unter Flüchtigkeit versteht man allgemein das Verdunstungsverhalten bzw. die Verdunstungsgeschwindigkeit von Stoffen, speziell von Flüssigkeiten. Da die Flüchtigkeit realer Flüssigkeiten von vielen Parametern abhängig ist, lässt sie sich kaum theoretisch berechnen. Zur Beurteilung der Flüchtigkeit bedient man sich daher empirisch ermittelter Maßzahlen. Die Verdunstungszahl (VD) ist eine Maßzahl für die Flüchtigkeit. Eine verwandte Kennzahl ist der Verdunstungskoeffizient (VK). Unter der Verdunstungszahl versteht man den Quotienten aus der Verdunstungszeit der zu prüfenden Flüssigkeit und derjenigen von Diethylether als Vergleichsflüssigkeit; als Prüftemperatur gilt 293 $\pm$ 2 K, die relative Luftfeuchtigkeit soll bei der Messung 65% $\pm$ 5% betragen (vgl. DIN 53170: 1991-08; 53249: 1995-01).

Flüchtige Substanzen im Sinne der vorliegenden Erfindung sind dementsprechend beispielsweise Ethanol (VD = 8,3), Isopropanol (VD = 1,7), n-Hexan (VD = 8,3), n-Heptan (VD = 3,7) und dergleichen mehr.

Besonders vorteilhaft im Sinne der vorliegenden Erfindung sind flüchtige Substanzen, deren Verdunstungszahl zwischen 3 und 12 liegt, insbesondere solche, deren Verdunstungszahl zwischen 5 und 10 liegt. Ganz besonders bevorzugt im Sinne der vorliegenden Erfindung ist Ethanol.

Bevorzugt stellen die erfindungsgemäßen Zubereitungen Emulsionen oder Hydrodispersionen dar. Im Gegensatz zur klassischen Emulsionen enthalten Hydrodispersionen aber nur sehr geringe Mengen an Emulgatoren (bis zu 2 Gew.-%) bzw. können sogar gänzlich frei von Emulgatoren sein.

In einer erfindungsgemäß besonders vorteilhaften Ausführungsform kann die erfindungsgemäße kosmetische Zubereitung in Form einer Öl-in-Wasser-Emulsion (OM/-Emulsion) vorliegen.

In diesem Fall werden der oder die O/W-Emulgatoren erfindungsgemäß vorzugsweise aus der folgenden Gruppe gewählt:

Glycerylstearat in Kombination mit Ceteareth-20 und/oder Ceteareth-25, Ceteareth-6 in Kombination mit Stearylalkohol, Cetylstearylalkohol in Kombination mit PEG-40-Ricinusöl und Natriumcetylstearylsulfat, Triceteareth-4 Phosphat, Glycerylstearat, Natriumcetylstearylsulfat, Lecithin Trilaureth-4 Phosphat, Laureth-4 Phosphat, Stearinsäure, Propylenglycolstearat SE, PEG-25-hydriertes Ricinusöl, PEG-54-hydriertes Ricinusöl und/oder PEG-6 Caprylsäure/Caprinsäureglyceride, Glyceryloleat in Kombination mit Propylenglycol, PEG-9-Stearat, PEG-20 Stearat, PEG-30-Stearat, PEG-40-Stearat, PEG-100-Stearat, Ceteth-2, Ceteth-20, Polysorbate-20, Polysorbate-60, Polysorbate-65 und/oder Polysorbate-100, Glycerylstearat in Kombination mit PEG-100 Stearat, Glycerylmyristat, Glyceryllaurat, PEG-40-Sorbitanperoleat, Laureth-4, Ceteareth-3 und/oder Isostearylglycerylether, Cetylstearylalkohol in Kombination mit Natrium Cetylstearylsulfat, Laureth-23 und/oder Steareth-2, Glycerylstearat in Kombination mit PEG-30 Stearat, PEG-40-Stearat, Glycol Distearat, PEG-22-Dodecyl Glycol Copolymer, Polyglyceryl-2-PEG-4-Stearat, Ceteareth-12, Ceteareth-20, Ceteareth-30, Methyl-glucosesesquistearat, Steareth-10 und/oder PEG-20-Stearat, Steareth-2 in Kombination mit PEG-8 Distearat, Steareth-21, Steareth-20, Isosteareth-20, PEG-45/ Dodecylglycol-Copolymer, Methoxy-PEG-22/Dodecylglycol-Copolymer, PEG-40-Sorbitanperoleat, PEG-40-Sorbitanperisostearat, PEG-20-Glycerylstearat, PEG-20-Glycerylstearat, PEG-8-Bienenwachs, Polyglyceryl-2-laurat, Isostearyldiglycerylsuccinat, Stearamidopropyl-PG-dimoniumchloridphosphat, Glycerylstearat SE, Ceteth-20, Triethylcitrat, PEG-20-Methylglucosesesquistearat, Glycerylstearatcitrat, Cetylphosphat, Cetearyl Sulfat, Sorbitansesquioleat, Triceteareth-4-Phosphat, Trilaureth-4-Phosphat, Polyglycerylmethylglucosedistearat, Kaliumcetylphosphat, Polyglyceryl-3 Methylglucose Distearate Isosteareth-10, Polyglyceryl-2-sesquiisostearat, Ceteth-10, Oleth-20 und/oder Isoceteth-20, Glycerylstearat in Kombination mit Ceteareth-20, Ceteareth-12, Cetylstearylalkohol und/oder Cetylpalmitat, Cetylstearylalkohol in Kombination mit PEG-20 Stearat, PEG-30-Stearat, PEG-40-Stearat und/oder PEG-100-Stearat.

Als erfindungsgemäß besonders vorteilhafte O/W-Emulgatoren werden Glycerylstearat, PEG-40 Stearat, PEG-100 Stearat Triglycerinmethylglucosedistearat, Polyglyceryl-3 Methylglucose Distearate, Polyethylenglycol(21)stearylether, Polyethylenglycol(2)stearylether, Cetearylglucosid, Glycerylstearatcitrat, Natriumcetearyl-sulfat, Cetearylalkohol, Stearinsäure und/oder Sorbitanstearat eingesetzt. Ferner vorteilhaft im Sinne der vorliegenden Erfindung ist Cetearylalkohol in Kombination mit PEG-40 hydriertes Rizinusöl und Natriumcetearylsulfat und Glycerylstearat.

Die Ölphase erfindungsgemäßer O/W-Emulsionen enthält vorteilhaft Ölkomponenten gewählt aus der Gruppe: Butylenglykoldicaprylat/-dicaprat, Dicaprylylether, $C_{12-15}$-Alkylbenzoat, $C_{18-38}$-Fettsäuretriglycerid, Dibutyladipat, Cyclomethicon. 2-Phenylethylbenzoat, Isopropyl Lauroyl Sarkosinat.

Die Menge der Ölkomponenten (eine oder mehrere Verbindungen) in erfindungsgemäßen O/W-Emulsionen beträgt vorzugsweise 0,1 bis 40 Gew.-%, besonders bevorzugt 1,0 bis 30 Gew.-%, insbesondere 5% bis 20 Gew.-%, bezogen auf das Gesamtgewicht der Zubereitung.

Die Ölphase von O/W-Emulsionen im Sinne der vorliegenden Erfindung kann vorteilhaft sowohl erfindungsgemäße Ölkomponenten mit einer Polarität kleiner oder gleich 35 mN/m als auch weitere Ölkomponenten enthalten, wobei das Verhältnis von erfindungsgemäßen zu weiteren Ölkomponenten bevorzugt wie 5 zu 1, besonders bevorzugt wie 10 zu 1 gewählt wird.

In einer zweiten erfindungsgemäß vorteilhaften Ausführungsform der vorliegenden Erfindung kann die erfindungsgemäße kosmetische Zubereitung in Form einer Hydrodispersion vorliegen.

Bei Hydrodispersionen, welche aus einer flüssigen Ölphase in einer äußeren wässrigen Phase bestehen, wird die Stabilität des Mehrphasensystems üblicherweise dadurch gewährleistet, dass in der wässrigen Phase mit Hilfe eines Gelbildners (Verdickungsmittels) ein Gelgerüst aufgebaut wird, in welchem die Lipidtröpfchen stabil suspendiert sind. Besonders bevorzugt ist es, wenn die erfindungsgemäßen Hydrodispersionen frei von Emulgatoren sind.

Das oder die Gelbildner werden erfindungsgemäß vorteilhaft aus einer oder mehreren der folgenden Gruppen gewählt:

- organische, natürliche Verbindungen, wie beispielsweise Agar-Agar, Carrageen, Tragant, Gummi arabicum, Alginate, Pektine, Polyosen, Guar-Mehl, Johannisbrotbaumkernmehl, Stärke, Dextrine, Gelatine, Casein,
- organische, abgewandelte Naturstoffe, wie z. B. Carboxymethylcellulose und andere Celluloseether, Hydroxyethyl- und -propylcellulose und mikrokristalline Cellulose dergleichen,
- organische, vollsynthetische Verbindungen, wie z. B. Polyacryl- und Polymethacryl-Verbindungen, Vinylpolymere, Polycarbonsäuren, Polyether, Polyimine, Polyamide, Polyurethane.

Bevorzugte Gelbildner im Sinne der vorliegenden Erfindung sind ferner Polyacrylate, d. h. Polymere auf Basis von Estern der Acrylsäure *(Polyacrylsäureester)* der allgemeinen Strukturformel

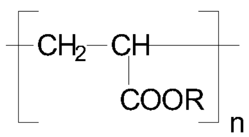

worin R für lineare, verzweigte oder cyclische, ggf. funktionelle Substituenten (z. B. Hydroxy-, Amin- oder Epoxid-Gruppen) enthaltende Alkyl-Reste steht, z. B. für Methyl-, Ethyl-, Isopropyl-, *tert*-Butyl-, Cyclohexyl-, 2-Ethylhexyl-, Dodecyl-, 2-Hydroxyethyl- und 2-Dimethylaminoethyl-.

Erfindungsgemäß besonders vorteilhafte Gelbildner sind (INCI-Bezeichnungen): Acrylates/C10-30 Alkyl Acrylate Crosspolymer, Carbomer, Ammonium Acryloyldimethyltaurate/VP Copolymer, Hydroxypropylcellulose, Hydroxyethylcellulose und Xanthan Gum.

Die Ölphase erfindungsgemäßer Hydrodispersionen enthält vorteilhaft Ölkomponenten gewählt aus der Gruppe: Butylenglykoldicaprylat/-dicaprat, Dicaprylylether, $C_{12-15}$-Alkylbenzoat, $C_{18-38}$-Fettsäuretriglycerid, Dibutyladipat, Cyclomethicon.

Die Menge der Ölkomponenten (eine oder mehrere Verbindungen) in erfindungsgemäßen Hydrodispersionen beträgt vorzugsweise 0,5 bis 35 Gew.-%, besonders bevorzugt 1,0 bis 25 Gew.-%, insbesondere 2,0 bis 15 Gew.-%, bezogen auf das Gesamtgewicht der Zubereitung.

Die Ölphase von Hydrodispersionen im Sinne der vorliegenden Erfindung kann vorteilhaft sowohl erfindungsgemäße Ölkomponenten mit einer Polarität kleiner oder gleich 35 mN/m als auch weitere Ölkomponenten enthalten, wobei das Verhältnis von erfindungsgemäßen zu weiteren Ölkomponenten bevorzugt wie 5 zu 1, besonders bevorzugt wie 10 zu 1 gewählt wird.

In einer dritten erfindungsgemäß vorteilhaften Ausführungsform der vorliegenden Erfindung kann die erfindungsgemäße kosmetische Zubereitung in Form einer Wasser-in-Öl-Emulsion (W/O-Emulsion) vorliegen. In diesem Fall ist es besonders bevorzugt, wenn der oder die W/O-Emulgatoren gewählt werden aus der Gruppe PEG-30 Dipolyhydroxystearat, Polyglyceryl-2-Dipolyhydroxystearat, Polyglyceryl-3-Diisostearat, PEG-40 Sorbitan Perisostearate.

Die Ölphase erfindungsgemäßer W/O-Emulsionen enthält vorteilhaft Ölkomponenten gewählt aus der Gruppe: Butylenglykoldicaprylat/-dicaprat, Paraffinum Liquidum, $C_{12-15}$-Alkylbenzoat, $C_{18-38}$-Fettsäuretriglycerid, Isopropylstearat, Cetyldimethicon, 2-Phenylethylbenzoat und/oder Isopropyl Lauroyl Sarkosinat.

Die Menge der Ölkomponenten (eine oder mehrere Verbindungen) in erfindungsgemäßen W/O-Emulsionen beträgt vorzugsweise 0,1 bis 40 Gew.-%, besonders bevorzugt 1,0 bis 30 Gew.-%, insbesondere 5% bis 20 Gew.-%, bezogen auf das Gesamtgewicht der Zubereitung.

Die Ölphase von W/O-Emulsionen im Sinne der vorliegenden Erfindung kann vorteilhaft sowohl erfindungsgemäße Ölkomponenten mit einer Polarität kleiner oder gleich 35 mN/m als auch weitere Ölkomponenten enthalten, wobei

das Verhältnis von erfindungsgemäßen zu weiteren Ölkomponenten bevorzugt wie 5 zu 1, besonders bevorzugt wie 3 zu 1 gewählt wird.

Zur Anwendung werden die erfindungsgemäßen Zubereitungen in der für Kosmetika üblichen Weise auf die Haut in ausreichender Menge aufgebracht.

Die kosmetischen Zubereitungen gemäß der Erfindung können ferner kosmetische Hilfsstoffe enthalten, wie sie üblicherweise in solchen Zubereitungen verwendet werden, z. B. Konservierungsmittel, Konservierungshelfer, Komplexbildner, Bakterizide, Parfüme, Substanzen zum Verhindern oder Steigern des Schäumens, Pigmente, die eine färbende Wirkung haben, Verdickungsmittel, anfeuchtende und/oder feuchthaltende Substanzen, Füllstoffe, die das Hautgefühl verbessern, oder andere übliche Bestandteile einer kosmetischen Formulierung wie Alkohole, Polyole, Polymere, Schaumstabilisatoren, Elektrolyte, organische Lösungsmittel oder Silikonderivate.

Ferner ist es vorteilhaft im Sinne der vorliegenden Erfindung, wenn die Zubereitungen einen oder mehrere Fettalkohole enthalten. Bevorzugter Fettalkohol ist der Cetylalkohol.

Die Gesamtmenge an einem oder mehreren Fettalkoholen in den fertigen kosmetischen Zubereitungen wird vorteilhaft aus dem Bereich von 0,1 bis 10,0 Gew.-%, bevorzugt 0,5 bis 6,0 Gew.-% gewählt, jeweils bezogen auf das Gesamtgewicht der Zubereitungen.

Die Wasserphase der erfindungsgemäßen Zubereitungen kann vorteilhaft übliche kosmetische Hilfsstoffe enthalten, wie beispielsweise

- Alkohole, insbesondere solche niedriger C-Zahl, vorzugsweise Ethanol und/oder Isopropanol, Diole oder Polyole niedriger C-Zahl sowie deren Ether, vorzugsweise Propylenglykol, 2-Methylpropan-1,3-diol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte,
- Polymere, Schaumstabilisatoren, Elektrolyte, Selbstbräuner (insbesondere Dihydroxyaceton) sowie
- besonders vorteilhaft ein oder mehrere Verdickungsmittel - insbesondere auch, wenn die erfindungsgemäße Zubereitung in Form einer Emulsion vorliegt. Das oder die Verdickungsmittel können vorteilhaft gewählt werden aus der Gruppe Aluminiumsilikate, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination. Weitere erfindungsgemäß vorteilhafte Verdicker sind solche mit der INCI-Bezeichnung Acrylates/C10-30 Alkyl Acrylate Crosspolymer (z. B. Pemulen TR 1, Pemulen TR 2, Carbopol 1328 von der Fa. NOVEON) sowie Aristoflex AVC (INCI: Ammonium Acryloyldimethyltaurate/VP Copolymer).

Erfindungsgemäß vorteilhaft können die erfindungsgemäßen Zubereitungen ferner einen oder mehrere Filmbildner enthalten. Filmbildner im Sinne der vorliegenden Erfindung sind Stoffe unterschiedlicher Zusammensetzung, die durch die folgende Eigenschaft charakterisiert sind: Löst man einen Filmbildner in Wasser oder anderen geeigneten Lösungsmitteln und trägt die Lösung dann auf die Haut auf, so bildet der Filmbildner nach dem Verdunsten des Lösemittels einen Film aus, der im wesentlichen dazu dient, die Lichtfilter auf der Haut zu fixieren und so die Wasserfestigkeit des Produktes zu steigern.

Es ist insbesondere von Vorteil, die Filmbildner aus der Gruppe der Polymere auf Basis von Polyvinylpyrrolidon (PVP)

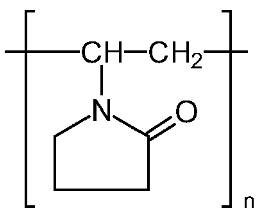

zu wählen. Besonders bevorzugt sind Copolymere des Polyvinylpyrrolidons, beispielsweise das PVP Hexadecen Copolymer und das PVP Eicosen Copolymer, welche unter den Handelsbezeichnungen Antaron V216 und Antaron V220 bei der GAF Chemicals Cooperation erhältlich sind.

Ebenfalls vorteilhaft sind weitere polymere Filmbildner, wie beispielsweise Natriumpolystryrensulfonat, welches unter der Handelsbezeichnung Flexan 130 bei der National Starch and Chemical Corp. erhältlich ist, und/oder Polyisobuten, erhältlich bei Rewo unter der Handelsbezeichnung Rewopal PIB1000. Weitere geeignete polymere Filmbildner sind z.B. Polyacrylamide (Seppigel 305), Polyvinylalkohole, PVP, PVP / VA Copolymere, Polyglycole, Acrylat/Octylacralymid Copolymer (Dermacryl 79). Ebenfalls vorteilhaft ist die Verwendung von Hydriertem Rizinusöl Dimerdilinoleat (CAS

646054-62-8, INCI Hydrogenated Castor Oil Dimer Dilinoleate), das bei der Firma Kokyu Alcohol Kogyo unter dem Namen Risocast DA-H erworben werden kann oder aber auch PPG-3 Benzylethermyristat (CAS 403517-45-3), das unter dem Handelsnamen Crodamol STS bei der Firma Croda Chemicals erworben werden kann.

Die erfindungsgemäßen Zubereitungen können ferner vorteilhaft eine oder mehrere Substanzen aus der Gruppe der Siloxanelastomere enthalten, beispielsweise um die Wasserfestigkeit und/oder den Lichtschutzfaktor der Produkte zu steigern. Vorteilhaft im Sinne der vorliegenden Erfindung sind Siloxanelastomere aus den folgenden Gruppen (a) und/oder (b):

(a) Siloxanelastomere, welche die Einheiten $R_2SiO$ und $RSiO_{1,5}$ und/oder $R_3SiO_{0,5}$ und/oder $SiO_2$ enthalten, wobei die einzelnen Reste R jeweils unabhängig voneinander Wasserstoff, C1-24-Alkyl (insbesondere Methyl, Ethyl, Propyl) oder Aryl (insbesondere Phenyl oder Tolyl, Alkenyl (insbesondere Vinyl) bedeuten und das Gewichtsverhältnis der Einheiten $R_2SiO$ zu $RSiO_{1,5}$ aus dem Bereich von 1 : 1 bis 30 : 1 gewählt wird;
(b) Siloxanelastomere, welche in Silikonöl unlöslich und quellfähig sind, die durch die Additionsreaktion eines

Organopolysiloxans (1), das siliciumgebundenen Wasserstoff enthält,
mit einem Organopolysiloxan (2), das ungesättigte aliphatische Gruppen enthält,
erhältlich sind,
wobei die verwendeten Mengenanteile so gewählt werden, dass die Menge des Wasserstoffes des Organopolysiloxans (1) oder der ungesättigten aliphatischen Gruppen des Organopolysiloxans (2)

- im Bereich von 1 bis 20 mol-% liegt, wenn das Organopolysiloxan nicht zyklisch ist und
- im Bereich von 1 bis 50 mol-% liegt, wenn das Organopolysiloxan zyklisch ist.

Vorteilhaft im Sinne der vorliegenden Erfindung liegen das oder die Siloxanelastomere in Form sphärischer Puder oder in Form von Gelen vor. Erfindungsgemäß vorteilhafte in Form sphärischer Puder vorliegende Siloxanelastomere sind die mit der INCI-Bezeichnung Dimethicone/Vinyl Dimethicone Crosspolymer, beispielsweise das von DOW CORNING unter der Handelsbezeichnungen DOW CORNING 9506 Powder erhältliche.

Ganz besonders bevorzugt ist es, wenn das Siloxanelastomer in Kombination mit unverzweigten bei Raumtemperatur flüssigen oder pastösen Silikonölen oder zyklischen Silikonölen oder deren Gemischen verwendet wird. Insbesondere vorteilhaft sind Organopolysiloxanelastomere mit der INCI-Bezeichnung Dimethicone/Polysilicone-11, ganz besonders die von der Grant Industries Inc. erhältlichen Gransil-Typen GCM, GCM-5, DMG-6, CSE Gel, PM-Gel, LTX, ININ Gel, AM-18 Gel und/oder DMCM-5.

Geeignete Konservierungsmittel im Sinne der vorliegenden Erfindung sind beispielsweise Formaldehydabspalter (wie z. B. DMDM Hydantoin, welches beispielsweise unter der Handelsbezeichnung Glydant ™ von der Fa. Lonza erhältlich ist), Iodopropylbutylcarbamate (z. B. die unter den Handelsbezeichnungen Glycacil-L, Glycacil-S von der Fa. Lonza erhältlichen und/oder Dekaben LMB von Jan Dekker), Parabene (d. h. p-Hydroxybenzoesäurealkylester, wie Methyl-, Ethyl-, Propyl- und/oder Butylparaben), Phenoxyethanol, Ethanol, Benzoesäure und dergleichen mehr. Üblicherweise umfasst das Konservierungssystem erfindungsgemäß ferner vorteilhaft auch Konservierungshelfer, wie beispielsweise Octoxyglycerin, Glycine Soja etc.

Obwohl die genannten Konservierungsmittel an sich in den erfindungsgemäßen Zubereitungen vorteilhaft eingesetzt werden können, sind ganz besonders bevorzugt solche Zubereitungen, welche frei von Parabenen und/oder frei von Formaldehydabspaltern sind.

Vorteilhafte Komplexbildner im Sinne der vorliegenden Erfindung sind beispielsweise EDTA, [S,S]-Ethylendiamindisuccinat (EDDS), welches beispielsweise unter der Handelsbezeichnung Octaquest von der Fa. Octel erhältlich ist, Pentanatrium-Ethylendiamintetramethylenphosphonat, welches z. B. unter dem Handelsnamen Dequest 2046 von der Fa. Monsanto erhältlich ist und/oder Iminodibersteinsäure, welche u. a. von der Fa. Bayer AG unter den Handelsnamen Iminodisuccinat VP OC 370 (ca. 30% ige Lösung) und Baypure CX 100 fest erhältlich ist.

Besonders vorteilhafte Zubereitungen werden ferner erhalten, wenn als Zusatz- oder Wirkstoffe Antioxidantien eingesetzt werden. Erfindungsgemäß enthalten die Zubereitungen vorteilhaft eines oder mehrere Antioxidantien. Als günstige, aber dennoch fakultativ zu verwendende Antioxidantien können alle für kosmetische Anwendungen geeigneten oder gebräuchlichen Antioxidantien verwendet werden.

Besonders vorteilhaft im Sinne der vorliegenden Erfindung können wasserlösliche Antioxidantien eingesetzt werden, wie beispielsweise Vitamine, z. B. Ascorbinsäure und deren Derivate.

Bevorzugte Antioxidantien sind ferner Vitamin E und dessen Derivate sowie Vitamin A und dessen Derivate.

Die Menge der Antioxidantien (eine oder mehrere Verbindungen) in den Zubereitungen beträgt vorzugsweise 0,001 bis 30 Gew.-%, besonders bevorzugt 0,05 bis 20 Gew.-%, insbesondere 0,1 bis 10 Gew.-%, bezogen auf das Gesamtgewicht der Zubereitung.

Sofern Vitamin E und/oder dessen Derivate das oder die Antioxidantien darstellen, ist vorteilhaft, deren jeweilige

Konzentrationen aus dem Bereich von 0,001 bis 10 Gew.-%, bezogen auf das Gesamtgewicht der Formulierung, zu wählen.

Sofern Vitamin A bzw. Vitamin-A-Derivate, bzw. Carotine bzw. deren Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Es ist insbesondere vorteilhaft, wenn die kosmetischen Zubereitungen gemäß der vorliegenden Erfindung kosmetische Wirkstoffe enthalten, wobei bevorzugte Wirkstoffe Antioxidantien sind, welche die Haut vor oxidativer Beanspruchung schützen können.

Weitere vorteilhafte Wirkstoffe im Sinne der vorliegenden Erfindung sind natürliche Wirkstoffe und/oder deren Derivate, wie z. B. alpha-Liponsäure, Phytoen, D-Biotin, Coenzym Q10, alpha-Glucosylrutin, Carnitin, Carnosin, natürliche und/oder synthetische Isoflavonoide, Kreatin, Taurin und/oder β-Alanin sowie 8-Hexadecen-1,16-dicarbonsäure (Dioic acid, CAS-Nummer 20701-68-2; vorläufige INCI-Bezeichnung Octadecendioic acid) und/oder Glycyrrhiza Inflata.

Erfindungsgemäße Rezepturen, welche z. B. bekannte Antifaltenwirkstoffe wie Flavonglycoside (insbesondere α-Glycosylrutin), Coenzym Q10, Vitamin E und/oder Derivate und dergleichen enthalten, eignen sich insbesondere vorteilhaft zur Prophylaxe und Behandlung kosmetischer Hautveränderungen, wie sie z. B. bei der Hautalterung auftreten (wie beispielsweise Trockenheit, Rauhigkeit und Ausbildung von Trockenheitsfältchen, Juckreiz, verminderte Rückfettung (z. B. nach dem Waschen), sichtbare Gefäßerweiterungen (Teleangiektasien, Cuperosis), Schlaffheit und Ausbildung von Falten und Fältchen, lokale Hyper-, Hypo- und Fehlpigmentierungen (z. B. Altersflecken), vergrößerte Anfälligkeit gegenüber mechanischem Stress (z. B. Rissigkeit) und dergleichen). Weiterhin vorteilhaft eignen sie sich gegen das Erscheinungsbild der trockenen bzw. rauen Haut.

Ferner vorteilhaft können die Zubereitungen gemäß der vorliegenden Erfindung auch Repellentien zum Schutz vor Mücken, Zecken und Spinnen und dergleichen enthalten. Vorteilhaft sind z. B. N,N-Diethyl-3-methylbenzamid (Handelsbezeichnung: Meta-delphene, "DEET"), Dimethylphtalat (Handelsbezeichnung: Palatinol M, DMP) sowie insbesondere 3-(N-n-Butyl-N-acetyl-amino)-propionsäureethylester (unter dem Handelsnamen Insekt Repellent® 3535 bei der Fa. Merck erhältlich). Ein weiterer vorteilhafter Repellent-Wirkstoff im Sinne der vorliegenden Erfindung ist der 1-Piperidincarboxylsäure 2-(2-hydroxyethyl)-1-methylpropylester (INN: Icaridin, CAS-Nummer: 119515-38-7, Elincs-Nummer: 423-210-8), der die folgende Struktur

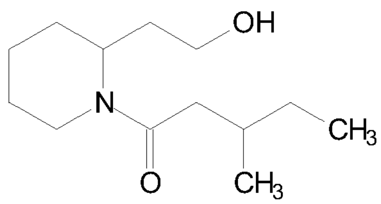

aufweist und als besonders wirksam gilt.

Die Repellentien können sowohl einzeln als auch in Kombination eingesetzt werden.

Auch sog. Moisturizer sind vorteilhaft zu verwenden. Als Moisturizer werden Stoffe oder Stoffgemische bezeichnet, welche kosmetischen Zubereitungen die Eigenschaft verleihen, nach dem Auftragen bzw. Verteilen auf der Hautoberfläche die Feuchtigkeitsabgabe der Hornschicht (auch transepidermal water loss (TEWL) genannt) zu reduzieren und/oder die Hydratation der Hornschicht positiv zu beeinflussen.

Vorteilhafte Moisturizer im Sinne der vorliegenden Erfindung sind beispielsweise Glycerin, Milchsäure und/oder Lactate, insbesondere Natriumlactat, Butylenglykol, Propylenglykol, Biosaccaride Gum-1, Glycine Soja, Ethylhexyloxyglycerin, Pyrrolidoncarbonsäure und Harnstoff.

Die erfindungsgemäßen kosmetischen Zubereitungen können ferner vorteilhaft, wenngleich nicht zwingend, Füllstoffe enthalten, welche z. B. die sensorischen und kosmetischen Eigenschaften der Formulierungen weiter verbessern und beispielsweise ein samtiges oder seidiges Hautgefühl hervorrufen oder verstärken. Vorteilhafte Füllstoffe im Sinne der vorliegenden Erfindung sind Stärke und Stärkederivate (wie z. B. Tapiocastärke, Distärkephosphat, Aluminium- bzw. Natrium-Stärke Octenylsuccinat und dergleichen), Pigmente, die weder hauptsächlich UV-Filter- noch färbende Wirkung haben (wie z. B. Bornitrid etc.) und/oder Aerosile® (CAS-Nr. 7631-86-9).

Die nachfolgenden Beispiele sollen die vorliegende Erfindung verdeutlichen, ohne sie einzuschränken. Die Zahlenwerte in den Beispielen bedeuten Gewichtsprozente, bezogen auf das Gesamtgewicht der jeweiligen Zubereitungen.

**Beispiele:**

**Beispiele für Gele**

| | 1 | 2 | 3 | 4 | 5 | 6 |
|---|---|---|---|---|---|---|
| Acrylates/Octylacrylamide Copolymer | 1,0 | 1,0 | 1,0 | 1,0 | 1,0 | 1,0 |
| Alohol Denat. | 50,0 | 62,0 | 59,2 | 70,0 | 70,0 | 69,0 |
| Butyl Methoxydibenzoylmethan | 4,5 | 4,5 | | | 2,5 | 4,5 |
| Butylene Glycol Dicaprylat/Dicaprat | | | 7,5 | | | 2 |
| C12-15 Alkyl Benzoate | 5,0 | 8,5 | 5,0 | 2 | 7,5 | |
| Phenylethylbenzoat | 3,0 | | | 2,5 | | |
| Cocoglycerid | | | | | | 2 |
| Tridecylsalicylat | 2 | 1,5 | 1,75 | 0,5 | 3 | 1 |
| Diethylamino Hydroxybenzoyl Hexyl Benzoate | | | 4,5 | 3,5 | | |
| Ethylhexyl Methoxycinnamat | 5 | 9,5 | | 6,5 | 6,5 | 9,5 |
| Ethylhexyl Salicylat | 4,5 | 4,5 | 4,5 | 4,5 | | |
| Homosalat | | | | | | 4,5 |
| Hydroxypropylcellulose | 2 | 0,8 | 1 | 0,8 | 0,5 | 0,8 |
| Octocrylen | 9,5 | 4,8 | 9,5 | 4,3 | 3,8 | |
| Ethylhexyltriazin | | | | | 2 | |
| Benzophenon-3 | 3 | | | | | |
| Drometrizol Trisiloxan | | | | 0,5 | 1,0 | |
| Bis-Ethylhexyloxyphenol Methoxy-phenyltriazin | | | | | | 1 |
| Vitamin E Acetat | | | | 0,5 | | 0,2 |
| Glycerin | 5 | | 3 | | | |
| Parfüm, Farbstoffe | q.s. | q.s. | q.s. | q.s. | q.s. | q.s. |
| Wasser | ad 100 | ad 100 | ad 100 | ad 100 | ad 100 | ad 100 |

**Beispiele für Sprays**

| | 1 | 2 | 3 | 4 | 5 | 6 |
|---|---|---|---|---|---|---|
| Acrylat/Octylacrylamid Copolymer | 1 | | | | 1 | 1 |
| VP/VA Copolymer | | 1,0 | | 0,5 | | |
| Alkohol Denat. | 42 | 57 | 60 | 53 | 35,5 | 43,5 |
| Bis-Ethylhexyloxyphenol Methoxyphenyl Triazin | | | | | 1,0 | |
| Uvasorb® K2A | 0,5 | | 1,0 | | | 3,0 |
| Butyl Methoxydibenzoylmethan | 4,5 | 2 | | 3 | 4,5 | |
| Cyclomethicon | 4,9 | 2 | 5 | 0,5 | 8,0 | 8 |
| Diethylamino Hydroxybenzoyl Hexyl Benzoat | | 2 | 3 | | | 4,5 |

(fortgesetzt)

| | 1 | 2 | 3 | 4 | 5 | 6 |
|---|---|---|---|---|---|---|
| Ethylhexyl Methoxycinnamat | 9,5 | | | 7 | 6,5 | 9,5 |
| Ethylhexyl Salicylat | 4,5 | 3,5 | 2 | 4 | 4,5 | 2,5 |
| Drometrizol Trisiloxan | | 0,5 | | | | 2,0 |
| Tridecylsalicylat | 0,5 | 2,5 | 4 | 7 | 10 | 3 |
| Homosalat | 9,5 | 5 | 3 | | 9,5 | 6,5 |
| Octocrylen | 9,5 | | 8 | | 7,5 | 9,5 |
| Merocyanin | | | 2 | | | |
| Butylen Glycol Dicaprylat/Dicaprat | | 9 | | | | |
| C12-15 Alkyl Benzoat | | 2 | | 2 | | |
| Phenylbenzoat | | | 7 | | 4 | |
| Diethylhexylnaphthalat | | 6 | | 3 | | |
| Isopropyl Lauroyl Sarkosinat | 2 | | 1 | | 3 | |
| Phenyl Trimethicone | 2 5 | | | | 1 2 | |
| Octyldodekanol | | | | 8 | | |
| Glycerin | 5 | 4 | 5 | 8 | 5 | 5 |
| Vitamin E Acetat | 0,1 | | 0,5 | | | |
| Parfüm, Farbstoffe | q.s. | q.s. | q.s. | q.s. | q.s. | q.s. |
| Wasser | ad 100 | ad 100 | ad 100 | ad 100 | ad 100 | ad 100 |

**O/W-Emulsionen**

| | 1 | 2 | 3 | 4 | 5 | 6 |
|---|---|---|---|---|---|---|
| Glyceryl Stearat Citrat | 2 | 2 | 3 | | | |
| Glyceryl Stearat SE | | | | 1 | 1 | 1,5 |
| Cetearyl Alkohol + PEG-40 Rizinusöl+ Natrium Cetearyl Sulfat | | | | 2,5 | 2,5 | 3 |
| Cetearylalkohol | | | 1 | 1 | | |
| Stearylalkohol | 0,5 | | | | | 2 |
| Myristylmyristat | 1,0 | 1 | | | 3 | |
| Acrylates/$C_{10-30}$ Alkyl Acrylat Cross-polymer | 0,1 | 0,2 | | | 0,1 | |
| Carbomer | | 0,2 | 0,3 | 0,2 | | |
| Xanthan Gum | 0,4 | | 0,2 | 0,2 | 0,3 | 0,4 |
| $C_{12-15}$ Alkyl Benzoat | | 3 | | | 5 | |
| 2-Phenylethylbenzoat | 5 | 2 | | | | |
| Butylenglycol Dicaprylat/Dicaprat | 5 | | | | 3 | 3 |
| Tridecylsalicylat | 1,5 | 2,5 | 0,25 | 5,9 | 7 | 15 |
| Dicaprylcaprat | 2 | 2 | | | 2 | 2 |

(fortgesetzt)

| | 1 | 2 | 3 | 4 | 5 | 6 |
|---|---|---|---|---|---|---|
| Cyclomethicon | | | 5 | 10 | | |
| Dimethicon | | | | 5 | | |
| PVP Hexadecen Copolymer | | 0,5 | | | | 1 |
| Propylenglykol | | | 1 | | 5 | 3 |
| Glycerin | 7,5 | 5 | 7 | 10 | 13 | 3 |
| Alkohol denat. | 2 | 3 | 1 | 7 | 0,5 | 10 |
| Titandioxid | 3 | | | 2 | | |
| Merocyanin | 2 | | 2 | | 3 | 3 |
| Phenylen-1,4-bis-(2-benzimidazyl)-3,3'-5,5'-tetrasulfonsäuresalz | 3 | 2 | | | | |
| Ethylhexyltriazin | 2,5 | 2 | | 1 | | |
| Phenylbenzimidazol Sulfonsäure | | | 2 | | | 1 |
| 4-Methoxyzimtsäure(2-ethylhexyl)ester | 9,5 | 5 | | 2 | | |
| Butyl Methoxydibenzoylmethan | | 1 | | | 3 | |
| Ethylhexylsalicylat | 2 | | 0,5 | 4 | 9,5 | |
| Octocrylen | | | | | 5 | 7 |
| Bis-Ethylhexyloxyphenol Methoxyphenyltriazin | | 1 | | 1 | | 1 |
| Vitamin E Acetat | 0,2 | 0,2 | 0,2 | 0,3 | 0,1 | 0,5 |
| $Na_2H_2$EDTA | 0,1 | 0,1 | 0,2 | 0,2 | 0,2 | 0,5 |
| Parfüm, Konservierungsmittel | q.s. | q.s. | q.s. | q.s. | q.s. | q.s. |
| Farbstoffe, usw. | q.s. | q.s. | q.s. | q.s. | q.s. | q.s. |
| Citronensäure, Natriumcitrat | q.s. | q.s. | q.s. | q.s. | q.s. | q.s. |
| Natriumhydroxid | q.s. | q.s. | q.s. | q.s. | q.s. | q.s. |
| Wasser | ad 100,0 | ad 100,0 | ad 100,0 | ad 100,0 | ad 100,0 | ad 100,0 |

| | 7 | 8 | 9 | 10 | 11 | 12 |
|---|---|---|---|---|---|---|
| Glycerylstearat | 2,5 | 2 | 1,2 | 1 | | |
| PEG-40 Stearat | 1 | | | | | |
| PEG-100 Stearat | | 2,5 | | | | |
| Ceteareth-20 | | | | | 1,0 | |
| Glyceryl Stearat Citrat | | | | | | 0,5 |
| Stearinsäure | | | 2,5 | 3 | | |
| Cetearylalkohol | 4,0 | | | 2 | | |
| Stearylalkohol | | 2 | 1 | | | |
| Cetylalkohol | | | 1 | 1 | | |
| Acrylates/$C_{10-30}$ Alkyl Acrylat Cross-polymer | | | | 0,2 | 0,2 | 0,4 |
| Carbomer | 0,1 | | 0,2 | | | |

(fortgesetzt)

| | 7 | 8 | 9 | 10 | 11 | 12 |
|---|---|---|---|---|---|---|
| Xanthan Gum | | 0,3 | | | | |
| $C_{12-15}$ Alkyl Benzoat | 5 | | | 2 | 5 | 5 |
| Vaseline | 5 | | 3 | | | |
| Butylenglycol Dicaprylat/Dicaprat | | 4 | 2 | | 9 | |
| Hydrogeniertes Polydecen | | | 3 | | 2 | |
| Caprylic/Capric Triglycerid | 1 | 3 | | 5 | | 5 |
| Cyclomethicon | | 5 | 2 | | | 10 |
| Metylpropandiol | 2 | | | | 3 | |
| Isopropyl Lauroyl Sarkosinat | | | | | | |
| Glycerin | 7,5 | 10 | 4 | 5 | 5 | |
| Ethanol | 1 | 3 | 0,5 | 10 | 4 | 8 |
| Butylen Glykol | | | 3 | | | |
| Titandioxid | 1 | | 0,5 | 2 | | |
| 2,4,6-Tribiphenyl-4-yl-1,3,5-triazin | | | 0,5 | 4 | | 6 |
| Dimethicodiethylbenzalmalonat | | | | 2 | | |
| Ethylhexylmethoxycinnamat | 4 | | | | 2 | |
| Phenylbenzimidazol Sulfonsäure | 1,5 | | | 2 | | 2 |
| Butyl Methoxydibenzoylmethan | 2,5 | | | 2 | | 3 |
| Isotridecylsalicylat | | | 1 | 3 | 5 | 2 |
| Tridecylsalicylat | 2 | 10 | 1 | 0,5 | 1 | |
| Octocrylen | | 5 | | | | 2 |
| Octylsalicylat | 3 | | | | 5 | |
| Homosalat | | | 4 | | 2 | |
| Drometrizol Trisiloxan | 0,5 | | | 1 | | 2 |
| Terephthaliden Dicampher Sulfonsäure | 0,75 | | | 0,5 | | 0,25 |
| Tapiokastärke | 1 | | 2,5 | | | 0,5 |
| Natrium-Stärke Octenylsuccinat | | | | 1 | | |
| $Na_2H_2EDTA$ | 0,1 | | | | | 0,5 |
| Parfüm, Konservierungsmittel | q.s. | q.s. | q.s. | q.s. | q.s. | q.s. |
| Natriumhydroxid | q.s. | q.s. | q.s. | q.s. | q.s. | q.s. |
| Wasser | ad 100,0 | ad 100,0 | ad 100,0 | ad 100,0 | ad 100,0 | ad 100,0 |

**W/O-Emulsionen**

| | 1 | 2 | 3 | 4 | 5 | 6 |
|---|---|---|---|---|---|---|
| Polyglyceryl-2 Dipolyhydroxystearat | 3 | 5 | 3 | | | |
| PEG-30 Dipolyhydroxystearat | | | 2 | 3 | 4 | 5 |

(fortgesetzt)

| | 1 | 2 | 3 | 4 | 5 | 6 |
|---|---|---|---|---|---|---|
| Natrium-Stärke Octenylsuccinat | 0,5 | 0,4 | 0,6 | 0,3 | 0,5 | 1 |
| Glycin | 0,3 | 0,3 | 0,5 | 0,4 | | |
| Alkohol | 2 | 5 | 2 | 0,5 | 8 | 1 |
| Magnesiumsulfat | 0,2 | 0,3 | 0,3 | 0,4 | 0,5 | 0,2 |
| $C_{12-15}$ Alkyl Benzoat | 5 3 | | | | 5 | |
| Triheptanoin | | 2 | | | | |
| Butylenglycol Dicaprylat/Dicaprat | 5 | | | | 3 | 3 |
| Dicaprylyl Ether | | | | | 2 | |
| Mineralöl | | 4 | 6 | 8 | | |
| Octyldodecanol | 2 | | | | | |
| Dicaprylcaprat | | 2 | | | 2 | 2 |
| Cyclomethicon | 5 | | 5 | 10 | | |
| Dimethicon | | | | 5 | | |
| Isohexadecan | | 1 | | | | |
| Butylenglycol | 5 | 8 | | | | 3 |
| Glycerin | 3 | 5 | 7 | 10 | 3 | 3 |
| Isotridecylsalicylat | 0,5 | 1 | | | 8 2 | |
| Tridecylsalicylat | | 1 | 2 | 4 | | 0,5 |
| Ethylhexylmethoxycinnamat | 5 | | 7 | | 5 | |
| Ethylhexyltriazin | 2 | 3 | 3 | | | 3 |
| Diethylhexylbutamidotriazin | | 1,5 | 1,5 | | | 1,5 |
| Butyl Methoxydibenzoylmethan | | 4 | | | | |
| 2-Phenylethylbenzoat | | | 2 | | 4 | |
| Isopropyl Lauroyl Sarkosinat | | | 1 | | 2 | |
| 2-(4'-Diethylamino-2'-hydoxybenzoyl)-benzoesäurehexylester | | | | 2 | | |
| Ethylhexylsalicylat | | | | | | |
| Titandioxid | 5 | 4 | 4 | | 3 4 | |
| Bis-Ethylhexyloxyphenol Methoxyphenyltriazin | 1 | 2 | 2 | | 2 3 | |
| Methylen Bis-Benztriazolyl Tetramethylbutylphenol | 2 | | 3 | | 1 | |
| Merocyanin | 2 | | 2 | 5 | 1 | |
| Vitamin E Acetat | 0,2 | 0,2 | 0,2 | 0,3 | 0,1 | 0,5 |
| $Na_2H_2EDTA$ | 0,1 | 0,1 | 0,2 | 0,2 | 0,2 | 0,5 |
| Parfüm, Konservierungsmittel | q.s. | q.s. | q.s. | q.s. | q.s. | q.s. |
| Wasser | ad 100,0 | ad 100,0 | ad 100,0 | ad 100,0 | ad 100,0 | ad 100,0 |

**Hydrodispersionen**

| | 1 | 2 | 3 | 4 | 5 | 6 |
|---|---|---|---|---|---|---|
| Glyceryl Stearat Citrat | | 0,40 | | | | |
| Sodium Carbomer | | | | | 0,30 | |
| Acrylates/C10-30 Alkyl Acrylate Cross-polymer | | | 0,30 | 0,40 | 0,10 | 0,10 |
| Ceteareth-20 | | | 1,00 | | | |
| Xanthan Gummi | 0,50 | | | 0,15 | | 0,50 |
| Dimethicon / Vinyl Dimethicon Cross-polymer | | | | 5,00 | | 3,00 |
| 2-(4'-Diethylamino-2'-hydoxybenzoyl)-benzoesäurehexylester | 0,25 | | | 0,50 | 2,00 | 1,50 |
| Butyl Methoxydibenzoylmethan | 2 | | 3,50 | | | |
| Bis-Ethylhexyloxyphenol Methoxypheny Triazin | | 2,00 | | 0,25 | | |
| Dinatrium Phenyl Dibenzimidazol Tetrasulfonat | 0,75 | | | | | 1,00 |
| Phenylbenzimidazol Sulfonsäure | | | 2,00 | | | |
| Ethylhexyl Methoxycinnamat | | | 7,00 | | 5,00 | 8,00 |
| Diethylhexyl Butamido Triazin | | | 2,00 | 2,00 | | |
| Ethylhexyl Triazin | 4,00 | 3,00 | | | 4,00 | |
| Octocrylen | | | | 10,00 | | 2,50 |
| Titandioxid | 0,50 | 2,00 | 1,00 | 2,00 | 3,00 | 1,00 |
| Merocyanin | | | | | | |
| Drometrizol Trisiloxan | | | | 1 | | 0,5 |
| Terephthaliden Dicampher Sulfonsäure | | | | 0,5 | | 0,75 |
| $C_{12-15}$ Alkyl Benzoat | 2,00 | | 2,50 | | | |
| Butylenglycol Dicaprylat/Dicaprat | 4,00 | | | | 6,00 | |
| Dicaprylyl Carbonat | | 3,00 | | | | |
| Dicaprylylether | | 2,00 | | | | |
| Cyclomethicon | | | | 7,50 | | |
| 2-Phenylethylbenzoat | | | 4 | | 2 | |
| Diethylhexylnaphthalat | 5 | | 6 | | | |
| Tridecylsalicylat | 2 | 3 | 1 | 5 | 3 | 0,5 |
| PVP Hexadecen Copolymer | 0,50 | | 0,50 | | 0,50 | 1,00 |
| Glycerin | 10,00 | 5,00 | 5,00 | | 5,00 | 15,00 |
| Butylenglycol | | 7,00 | | | | |
| Glycin Soja | | | | 1,00 | | |
| Vitamin E Acetat | 0,50 | 0,25 | 0,50 | 0,25 | 0,75 | 1,00 |
| $\alpha$ - Glycosylrutin | | | | | 0,25 | |
| Trinatrium EDTA | | 1,00 | 1,00 | 0,10 | 0,20 | |
| Ethanol | 3,00 | 10,00 | 4,00 | 3,50 | 0,5 | 1,00 |

(fortgesetzt)

| | 1 | 2 | 3 | 4 | 5 | 6 |
|---|---|---|---|---|---|---|
| Konservierungsmittel | q.s. | q.s. | q.s. | q.s. | q.s. | q.s. |
| Parfüm, Farbstoffe, | q.s. | q.s. | q.s. | q.s. | q.s. | q.s. |
| Wasser | ad 100 | ad 100 | ad 100 | ad 100 | ad 100 | ad 100 |

## Patentansprüche

1. Kosmetische Lichtschutzzubereitung, **dadurch gekennzeichnet, dass** sie

    a) eine oder mehrere öllösliche UV-Filtersubstanzen,
    b) eine oder mehrere Ölkomponenten mit einer Polarität ≤ 35 mN/m,
    c) einen oder mehrere Salicylatester, welcher durch die Strukturformel

    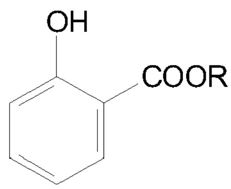

    **gekennzeichnet** ist, worin R einen unverzweigten Alkylrest mit 5 bis 16 Kohlenstoffatomen oder einen verzweigten Alkylrest mit 9 bis 20 Kohlenstoffatomen darstellt,
    d) eine oder mehrere flüchtige Substanzen, deren Verdunstungszahl zwischen 3 und 12 liegt und
    e) Wasser

    enthält.

2. Zubereitung nach Anspruch 1, **dadurch gekennzeichnet, dass** sie eine UVA-Balance von mehr als 22 aufweist.

3. Zubereitung nach einem der vorhergehenden Ansprüche, **dadurch gekennzeichnet, dass** sie eine UVA-Balance von mehr als 40 aufweist.

4. Zubereitung nach einem der vorhergehenden Ansprüche, **dadurch gekennzeichnet, dass** die Ölkomponenten mit einer Polarität ≤ 35 mN/m aus der Gruppe der Substanzen mit den INCI-Bezeichnungen Octyldodecanol, C12-15 Alkyl Benzoate, Butylene Glycol Dicaprylate/Dicaprate, Cyclomethicon, 2-Phenylethylbenzoat, Isopropyl Lauroyl Sarkosinat gewählt werden, jeweils einzeln oder in Kombinationen miteinander.

5. Zubereitung nach einem der vorhergehenden Ansprüche, **dadurch gekennzeichnet, dass** Salicylatester gewählt werden, für die R einen unverzweigten Alkylrest darstellt.

6. Zubereitung nach Anspruch 5, **dadurch gekennzeichnet, dass** der unverzweigte Alkylrest 10 bis 16 Kohlenstoffatome aufweist.

7. Zubereitung nach einem der vorhergehenden Ansprüche, **dadurch gekennzeichnet, dass** die Verdunstungszahl der flüchtigen Substanzen (eine oder mehrere Verbindungen) zwischen 5 und 10 liegt.

8. Zubereitung nach einem der vorhergehenden Ansprüche, **dadurch gekennzeichnet, dass** sie eine Emulsion oder Hydrodispersion darstellt.

9. Zubereitung nach Anspruch 8, **dadurch gekennzeichnet, dass** sie ein feindisperses System vom Typ Öl-in-Wasser darstellt.

**10.** Zubereitung nach einem der Ansprüche 8 oder 9, **dadurch gekennzeichnet, dass** sie ein oder mehrere Verdickungsmittel enthält.

**11.** Zubereitung nach Anspruch 10, **dadurch gekennzeichnet, dass** der oder die Verdicker aus der Gruppe Acrylates/C10-30 Alkyl Acrylate Crosspolymer, Carbomer, Ammonium Acryloyldimethyltaurate/VP Copolymer, Hydroxypropylcellulose, Hydroxyethylcellulose und Xanthan Gum gewählt werden.

**12.** Zubereitung nach einem der vorhergehenden Ansprüche, **dadurch gekennzeichnet, dass** der Gehalt an Octylmethoxycinnamat - bezogen auf das Gesamtgewicht der Zubereitung - zwischen 0,1 und 2 Gew.-% liegt.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Nicht-Patentliteratur


- **WENDEL V. et al.** The influence of pre-irradiation on the predictability of in vivo UVA protection with a new in vitro method. *Photodermatol Photoimmunol Photomed,* 2003, vol. 19, 93-97